# EUROPÄISCHE PATENTSCHRIFT

(11) **EP 3 240 489 B1**
(45) Veröffentlichungstag und Bekanntmachung des Hinweises auf die Patenterteilung: **22.07.2020**
(21) Anmeldenummer: 15816201.6
(22) Anmeldetag: 23.12.2015
(51) Int. Cl.: A61B 17/16, B25F 5/00, B25F 5/02

(54) **MEDIZINISCHER HANDGRIFF MIT PNEUMATISCHER SICHERHEITSKUPPLUNG**
MEDICAL HANDLE WITH PNEUMATIC SAFETY COUPLING
POIGNÉE À USAGE MÉDICAL POURVU D'UN ACCOUPLEMENT DE SÉCURITÉ PNEUMATIQUE

(30) Priorität: 29.12.2014 DE 102014119679
(43) Veröffentlichungstag der Anmeldung: 08.11.2017
(73) Patentinhaber: Aesculap AG, 78532 Tuttlingen (DE); Rotomed AG, 4512 Bellach (CH)
(72) Erfinder: HEINE, Wolfgang, 78194 Immendingen (DE); BLUST, Edgar, 78126 Königsfeld (DE); MÜLLER, Thomas, 4512 Bellach (CH); LENZENHUBER, Frederick, 78532 Tuttlingen (DE); HILDEBRAND, Anette, 78086 Brigachtal (DE); HERMLE, Simone, 78050 VS-Villingen (DE); MATTES, Uwe, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2015/081208
(87) Internationale Veröffentlichungsnummer: WO 2016/107814

(56) Entgegenhaltungen:
- EP-A2- 1 302 282
- EP-A2- 2 532 316
- US-A- 3 752 241
- US-A1- 2004 099 318
- US-B1- 6 520 976

## Beschreibung

Die vorliegende Erfindung betrifft einen Handgriff medizinischer Instrumente mit integrierter pneumatischer/hydraulischer Sicherheitskupplung gemäß dem Oberbegriff des Patentanspruchs 1.

### Hintergrund der Erfindung

Medizinische, insbesondere chirurgische Instrumente betreffen u. a. solche Instrumente, die mit motorisch angetriebenen Werkzeugen wie Fräser, Bohrer, Schrauber, etc. bestückt oder bestückbar sind. Der Antrieb, beispielsweise zur Dreh- oder Hubbewegung des Werkzeugs, kann durch einen elektrischen Motor, hydraulisch oder pneumatisch erfolgen. Insbesondere im letzteren Fall wird das Instrument an eine Druckluftquelle angeschlossen, mittels welcher beispielsweise eine Turbine innerhalb des Instruments oder als separate Motoreinheit wahlweise und ggf. gesteuert Druckluft - beaufschlagt wird, deren Drehung dann direkt oder indirekt über ein Getriebe auf das Werkzeug übertragen wird.

Derartige pneumatisch oder hydraulisch betriebene Instrumente sind indessen nicht permanent mit der Druckmittelquelle fest verbunden sondern können beliebig an die Druckmittelquelle angeschlossen oder von dieser getrennt werden. Hierfür sind Kupplungen erforderlich, mittels denen die Instrumente wahlweise mit der Druckmittelquelle pneumatisch oder hydraulisch verbindbar sind. An diese Kupplungen werden insbesondere in der chirurgischen Medizin große sicherheitstechnische Anforderungen gestellt, um Verletzungen von Patienten auszuschließen und die Handhabung der Instrumente für den Chirurgen zu erleichtern.

### Stand der Technik

Aus dem Stand der Technik sind medizinische, vorzugsweise chirurgische Instrumentensysteme mit pneumatischem Werkzeugantrieb bekannt, wie sie auch von der vorliegenden Anmelderin selbst seit Jahren vertrieben werden.

Derartige Instrumentensysteme beinhalten in der Regel eine Druckmittelquelle (nachfolgend der Einfachheit halber als Druckluftquelle bezeichnet), an die ein vorzugsweise flexibler Druckschlauch angeschlossen oder anschließbar ist. Am stromabwärtigen, freien Ende des Druckschlauchs ist eine als Betätigungs- und/oder Steuereinheit ausgebildete Handhabe in Form eines Handgriffs vorzugsweise fest (d.h. unlösbar) montiert, an dessen distalem, freiem Ende wiederum eine Kupplung für ein medizinisches/chirurgisches Instrument ausgebildet ist. Die Kupplung übernimmt dabei die mechanische Verbindung zwischen dem Handgriff und dem ausgewählten Instrument und gleichzeitig den pneumatischen Anschluss eines Instrumenten-internen oder separaten Pneumatikmotors (z.B. Turbine) an eine Handgriff-interne pneumatische Betätigungs- und/oder Steuereinrichtung.

Die Betätigungs- und/oder Steuereinrichtung besteht aus einem manuell betätigbaren Stellglied vorzugsweise in Form eines am Handgriff schwenkbar gelagerten Betätigungshebels und/oder mindestens eines Druckkopfs, wobei das Stellglied mechanisch direkt oder pneumatisch/hydraulisch vorgesteuert indirekt auf einen Regel-/Steuerventil-Mechanismus innerhalb des Handgriffs einwirkt, der in Abhängigkeit des aktuellen Betätigungszustands eine Druckluftverbindung zum Instrument freigibt oder sperrt. Die Freigabe kann entweder nach dem Ein-Aus-Prinzip oder dosiert entsprechend dem Betätigungsgrad des Stellglieds erfolgen.

Da der Handgriff in der Regel bereits an der Druckluftquelle angeschlossen ist und somit unter Druck steht, bevor ein ausgewähltes Instrument am Handgriff angekoppelt wird, besteht grundsätzlich die Gefahr, dass das Stellglied unbeabsichtigt betätigt wird und dabei Druckluft unkontrolliert entweichen kann. Außerdem kann das Problem auftreten, dass bei unbeabsichtigt betätigtem Stellglied das aktuell ausgewählte Instrument angekoppelt wird, welches sodann unkontrolliert und unbeabsichtigt mit Druckluft beaufschlagt und somit angetrieben wird.

Daher sieht der bekannte Stand der Technik am/im Handgriff eine separate Druckluftsicherung vor, die nach dem Ankuppeln des Instruments bzw. eines vorgeschalteten Motors an den Handgriff (und damit an den Druckschlauch - Druckluft liegt an) manuell freigeschaltet werden muss, um die Funktion des Stellglieds freizugeben. Eine solche Druckluftsicherung kann beispielsweise ein Stellglied - Riegel sein, der mittels eines am vorzugsweise hebelförmigen Stellglied gelagerten Schiebers betätigbar ist, um das Stellglied zu ver- und/oder zu entriegeln.

Obgleich durch diese zusätzliche Druckluftsicherung die vorstehend genannten Nachteile und Gefahren vermieden werden können, verkompliziert sich ggf. die Handhabung des Instruments im chirurgischen Betrieb.

In der Druckschrift US 6,520,976 B1 wird ein zweiteiliges medizinisches Instrument offenbart, bei welchem ein Kupplungsabschnitt eines ersten Teils unmittelbar mit einer Ventileinheit eines zweiten Teils zusammenwirkt, um eine Freischaltung der Ventileinheit zu erwirken, wenn die beiden Teile des medizinischen Instruments gekoppelt werden.

Einen ersten Teil und einen zweiten Teil aufweisende Instrumente, bei welchen während des Koppelns der zwei Teile ein Kupplungsabschnitt des ersten Teils zur Freischaltung einer Ventileinheit des zweiten Teils unmittelbar mit der Ventileinheit zusammenwirkt, sind des Weiteren bekannt EP 1 302 282 A2, US 3,752,241 A und US 2004/0099318 A1.

### Kurzbeschreibung der Erfindung

Angesichts des vorstehend beschriebenen Stands der Technik ist es die generelle Aufgabe der vorliegenden Erfindung, den vorstehend beschriebenen medizinischen Handgriff (mit den hierzu bereits genannten technischen Merkmalen sowie das medizinischen Instrumentensystem mit Handgriff und Instrument und/oder Antriebsmotor - Einheit für das Instrument so weiter zu bilden, dass hiermit eine hohe Funktions- und/oder Betriebssicherheit gewährleistet wird, ohne dass sich die Handhabung übergebührlich erschwert.

Diese Aufgabe wird durch einen medizinischen Handgriff medizinisch/chirurgischer Instrumente mit den (zusätzlichen) Merkmalen des Anspruchs 1 und ein medizinisch/chirurgisches Instrumentensystem mit den (zusätzlichen) Merkmalen des Anspruchs 8 gelöst. Vorteilhafte Ausgestaltungen der Erfindung sind Gegenstand der Unteransprüche.

Der Kerngedanke der Erfindung besteht darin, dass die aus einem männlichen Teil, vorzugsweise auf Seiten des chirurgischen Instruments bzw. der Motoreinheit und einem weiblichen Teil, vorzugsweise auf Seiten des Handgriffs bestehende Kupplung im Bereich des Handgriffs mit dem Handgriff - internen Ventilmechanismus bzw. Regel-/Steuerventil-Mechanismus zusammenwirkt, der beim oder durch den mechanischen Kopplungsvorgang selbst (automatisch/zwangsläufig) geöffnet wird, wohingegen im entkoppelten Zustand der Ventilmechanismus bzw. Regel-/Steuerventil-Mechanismus geschlossen ist. Auf diese Weise ist die Anordnung einer separat zu betätigenden Druckluftsicherung unnötig und damit die Handhabung vereinfacht. Gleichzeitig wird die Funktions- und/oder Betriebssicherheit verbessert.

Konkreter ausgedrückt lässt sich der vorstehende Kerngedanke der vorliegenden Erfindung (Erfindungskonzept) durch zwei Maßnahmen technisch umsetzen:
Als erste Maßnahme ist das (weibliche) Kupplungsteil auf Seiten des Handgriffs mit einer ersten Ventileinrichtung des Ventilmechanismus bzw. Regel-/Steuerventil-Mechanismus versehen bzw. wirkverbunden, die so ausgelegt ist, dass sie spätestens durch/beim druckfesten Verriegeln der Kupplung (über einen ersten Bewegungsübertragungszug) zwangsweise geöffnet wird. Als zweite Maßnahme wirkt das (weibliche) Kupplungsteil auf Seiten des Handgriffs mit einer zweiten, manuell zu betätigenden Ventileinrichtung des Ventilmechanismus bzw. Regel-/Steuerventil-Mechanismus des Handgriffs (über einen zweiten Bewegungsübertragungszug) zusammen, dergestalt, dass die zweite Ventileinrichtung nur im gekoppelten und vorzugsweise verriegelten Zustand funktionsfähig ist und im entkoppelten Zustand (bei abgenommenem Instrument/Motoreinheit) nicht funktionsfähig ist und daher nicht mehr manuell geöffnet werden kann.

Letzteres lässt sich vorzugsweise dadurch erreichen, indem insbesondere durch/mit Verriegeln der Kupplung auf Seiten des Handstücks gleichzeitig (zwangsläufig/automatisch) die Funktionsfähigkeit des vorzugsweise hebelförmigen Stellglieds hergestellt wird bzw. durch/mit Entriegeln der Kupplung und/oder Abkoppeln des Instruments/Motoreinheit das Stellglied (zwangsläufig/automatisch) außer Funktion gesetzt wird.

Eine einfache konstruktive Lösung der beiden vorstehend genannten Maßnahmen sieht prinzipiell vor, ein bewegbar am Handgriff gelagertes, manuell betätigbares Kupplungs-Verriegelungselement (mechanisch über den ersten Bewegungsübertragungszug) mit der ersten Ventileinrichtung und (mechanisch über den zweiten Bewegungsübertragungszug) mit dem Stellglied der zweiten Ventileinrichtung wirkzuverbinden, sodass ein manuelles Betätigen des KupplungsVerriegelungselements automatisch ein Öffnen/Schließen der ersten Ventileinrichtung bewirkt bzw. eine entsprechende Wirkung auf die Funktionsfähigkeit des Stellglieds ausübt, beispielsweise indem das Stellglied in eine Position bewegt wird, in welcher die Wirkverbindung mit einem Ventil unterbrochen bzw. hergestellt ist oder eine (mechanische) Sperre in den oder aus dem Betätigungsweg des Stellglieds gefahren wird.

Der erste Bewegungsübertragungszug kann beispielsweise einen axial verschiebbaren Betätigungsstift aufweisen, der vom Kupplungsverriegelungselement aktuierbar ist und in Abhängigkeit der Betätigungssituation des Kupplungsverriegelungselements die erste Ventileinrichtung zwischen ihrer Auf - Zu - Position hin- und her schaltet. Der zweite Bewegungsübertragungszug kann beispielsweise eine axial verschiebbare Hülse oder Schubstange sein, die mit der Lagerung des Stellglieds mechanisch gekoppelt ist, und so die Lagerung des Stellglieds in Abhängigkeit der Betätigungssituation des Kupplungsverriegelungselements axial verschiebt.

Diese konstruktive Ausgestaltung bietet zusätzlich die Möglichkeit, das Kupplungs-Verriegelungselement mit mehreren (axialen) Betätigungspositionen auszustatten, beispielsweise eine ersten Betätigungsposition (Entriegelungsposition) für ein Entkoppeln des Handgriffs und des Instruments/Motoreinheit, eine zweite Betätigungsposition (aktive Verriegelungsposition) für ein Verriegeln der Kupplung bei gleichzeitigem Öffnen der ersten Ventileinrichtung und in Funktion setzen der zweiten Ventileinrichtung und einer dritten Betätigungsposition (passive Verriegelungsposition) für ein Verriegeln der Kupplung bei gleichzeitigem Öffnen der ersten Ventileinrichtung und außer Funktion setzen der zweiten Ventileinrichtung. Die Bereitstellung der dritten Betätigungsposition bietet wiederum die Möglichkeit der Anordnung eines Art Not - Aus - Schalters, bei dessen Betätigung das Kupplungs - Verriegelungselement vorzugsweise selbsttätig (aufgrund einer geeigneten Vorspannung) in die dritte Betätigungsposition (liegt vorzugsweise im Betätigungsweg zwischen der ersten und zweiten Betätigungsposition) verlagert wird.

Das Kupplungs-Verriegelungselement kann bevorzugt eine Überwurfhülse sein, welche in Verriegelungsposition kupplungsseitige Rast- oder Klemmelemente radial übergreift und diese in verriegelter Stellung fixiert.

### Figurenbeschreibung

Die Erfindung wird nachstehend anhand eines bevorzugten Ausführungsbeispiels unter Bezugnahme auf die begleitenden Figuren näher erläutert.

Es zeigen:
Fig. 1a die Seitenansicht eines medizinischen/chirurgischen Instrumentensystems gemäß einem bevorzugten Ausführungsbeispiel der Erfindung insbesondere eines medizinischen Handgriffs mit Betätigungselement/Stellglied und integriertem Regel-/Steuermechanismus sowie eines chirurgischen Instruments bzw. einer Motoreinheit derselben im entriegelten sowie entkoppelten Zustand gemäß einem bevorzugten Ausführungsbeispiel der vorliegenden Erfindung,
Fig. 1b die Draufsicht auf den Handgriff gemäß der Fig. 1a,
Fig. 2 die Längsschnittansicht des medizinischen/chirurgischen Instrumentensystems nach Fig. 1a,
Fig. 3a und 3b eine vergrößerte Längsschnittansicht einer entriegelten sowie entkoppelten Kupplung für das fluidische und gleichzeitig mechanische Koppeln von Handgriff und Instrument/Motoreinheit,
Fig. 4 die Längsschnittansicht des medizinischen/chirurgischen Instrumentensystems nach Fig. 1a im gekoppelten sowie verriegelten Zustand mit geöffneter erster Ventileinrichtung und in Funktion gesetzter zweiter Ventileinrichtung,
Fig. 5a und 5b eine vergrößerte Längsschnittansicht der gekoppelten und verriegelten Kupplung für das fluidische und gleichzeitig mechanische Kuppeln des Handgriffs mit dem Instrument/Motoreinheit gemäß Fig. 4,
Fig. 6 eine vergrößerte Teil-Längsschnittansicht insbesondere des Handgriffs bei gekoppelter und verriegelter Kupplung mit geöffneter erster Ventileinrichtung und in Funktion gesetzter zweiter Ventileinrichtung,
Fig. 7 eine vergrößerte Teil-Längsschnittansicht insbesondere des Handgriffs bei gekoppelter und verriegelter Kupplung mit geöffneter erster Ventileinrichtung und außer Funktion gesetzter zweiter Ventileinrichtung (3. Betätigungsposition bzw. Not-Aus-Position) und
Fig. 8 das medizinische/chirurgische Instrumentensystem mit allen notwendigen Elementen.

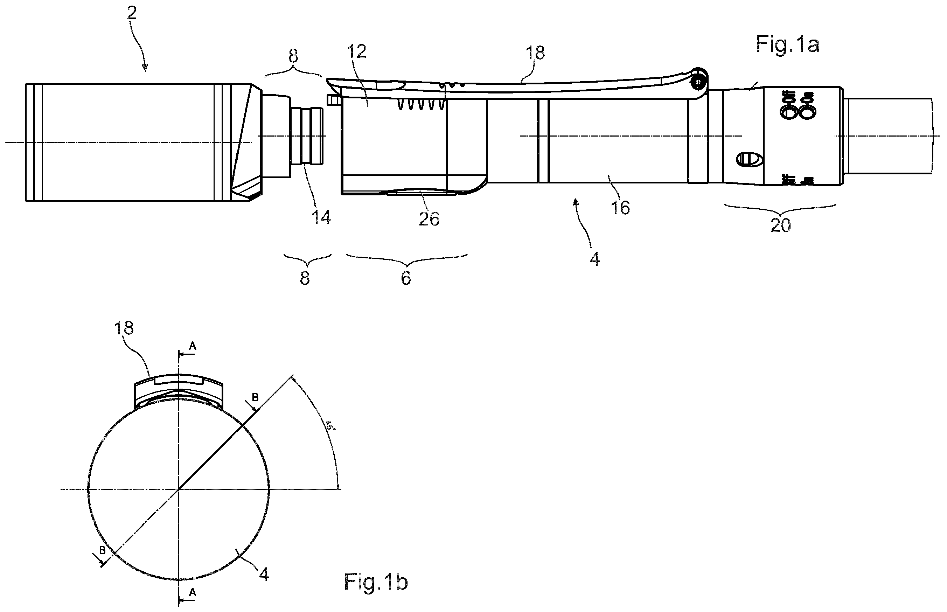

Gemäß der Fig. 1a, 1b, 2 und 8 hat ein medizinisches/chirurgisches Instrumentensystem allgemein ein medizinisches Instrument 1 entweder mit einem internen Hydraulik-/Pneumatikmotor oder mir einer externen/separaten Motoreinheit der Hydraulik-/Pneumatikbauart 2 und einen medizinischen Handgriff 4 für pneumatisch oder hydraulisch angetriebene medizinische Instrumente oder Motoreinheiten. Der Handgriff 4 hat an seinem distalen Ende ein vorzugsweise weibliches Kupplungsteil 6 zur mechanischen und pneumatisch-/hydraulischen Ankupplung eines entsprechenden, vorzugsweise männlichen Kupplungsteils 8 auf Seiten des Instruments 1 oder der vorgelagerten Motoreinheit 2 und einen internen, manuell betätigbaren Ventilmechanismus bzw. Regel-/Steuerventil-Mechanismus 10 zur wahlweisen Druckbeaufschlagung des daran angekuppelten medizinischen Instruments 1 oder dessen vorgelagerter Motoreinheit 2. Der Ventilmechanismus 10 hat u. a. eine Sicherungseinrichtung bzw. Sicherungsfunktion zur Verhinderung einer Druckbeaufschlagung im Fall des entriegelten und/oder abgekoppelten medizinischen Instruments 1 oder dessen Motoreinheit 2.

Das auf Seiten des Handgriffs 4 bestehende (weibliche) Kupplungsteil 6 ist so aufgebaut, dass es mit dem Ventilmechanismus 10 (mechanisch) zusammenwirkt, derart, dass dieser beim oder durch den mechanischen Kopplungsvorgang insbesondere beim oder durch Verriegeln des bereits gekoppelten Kupplungsteils 6 mittels eines manuell betätigbaren Kupplungs - Verriegelungselements 12 automatisch/zwangsläufig geöffnet und wird, wohingegen im entriegelten und/oder entkoppelten Zustand der Ventilmechanismus 10 geschlossen und ist.

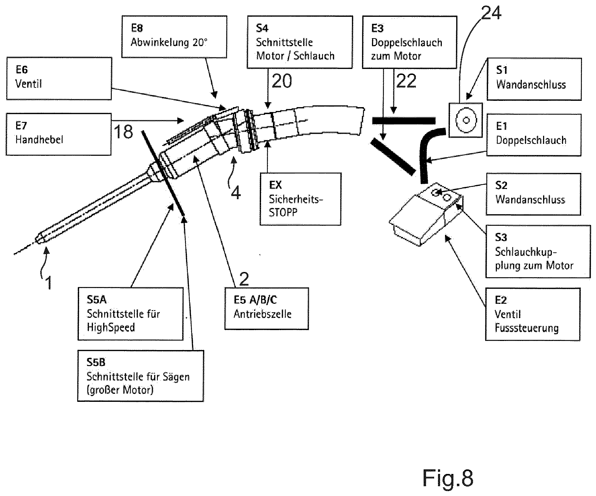

An dieser Stelle werden die bis dato verwendeten technischen Begriffe wie folgt definiert:
- Das medizinische/chirurgische Instrument 1 betrifft gemäß Fig. 8 jenen Abschnitt des Instrumentensystems, in welchem ein Werkzeug, beispielsweise ein Bohrer, Fräser, Schrauber etc. gelagert ist. Ggf. nimmt das Instrument 1 auch ein Getriebe auf. Ferner kann das Instrument 1 eine mechanische Kupplung für das Anschließen einer separaten Motoreinheit aufweisen. Alternativ hierzu kann das Instrument 1 bereits mit einem Pneumatik-/Hydraulikmotor intern/integral versehen sein.
- Die Motoreinheit 2 betrifft einen in der Regel patronenartigen Motorblock, welcher dem Instrument 1 vorgelagert ist. Der Motorblock kann als separate Einheit ausgebildet und wahlweise mit dem Instrument 1 oder einem dazwischen angeordneten Getriebe (nicht weiter gezeigt) mechanisch gekoppelt werden.
- Der Motor kann eine Turbine aufweisen, die per Druckluft in Rotation versetzbar ist. Es kann aber auch ein Pneumatik-/Hydraulikzylinder für eine Axialbewegung bei Druckbeaufschlagung als Motor vorgesehen sein.
- Der Handgriff 4 betrifft jenen Abschnitt des Instrumentensystems, der zur manuellen Betätigung des Instruments 1 sowie zum Halten des Instruments 1 vorgesehen ist. Er ist mit entsprechenden Betätigungseinrichtungen wie Stellglieder zur (Ein - Aus/Betätigungsbetragsabhängigen) Aktuierung eines internen/integralen Ventilmechanismus 10, Verriegelungselementen 12 für die fluidische und mechanische Kopplung zum Instrument/Motoreinheit und/oder Not - Aus - Schalter versehen.
- Der entkoppelte Zustand ist jener Zustand, in welchem der Handgriff 4 vom Instrument/Motoreinheit 1 bzw. 2 getrennt ist.
- Der entriegelte Zustand ist jener Zustand, in welchem der Handgriff 4 mit dem Instrument/Motoreinheit 1 bzw. 2 zwar gekoppelt, die Kupplung aber noch nicht verriegelt und damit wieder leicht entkoppelbar ist.
- Der verriegelte Zustand ist jener Zustand, in welchem die gekoppelten Kupplungsteile verriegelt und die Kupplung damit kraftbelastbar ist.

In der Fig. 1a ist das medizinische/chirurgische Instrumentensystem vorzugsweise der Pneumatikbauart beispielhaft mit einer separaten Motoreinheit 2 dargestellt, wobei das Instrument selbst nicht gezeigt ist. Hierzu wird aber auf die Fig. 8 verwiesen, in welcher das Instrument 1 in Form eines Rotationsfräsers gezeigt ist, der bereits mit einer separaten Motoreinheit 2 als Antriebszelle gekoppelt ist.

In der Fig. 1a ist das männliche Kopplungsteil 8 auf Seiten der Motoreinheit 2 erkennbar, das in Form eines Stutzens an der proximalen Stirnseite der Motoreinheit 2 axial vorragt. Der Stutzen bildet intern einen Fluidkanal, der in Richtung einer nicht weiter gezeigten Motorturbine einen Fluidauslass hat. Am Außenumfang des Stutzens ist wenigstens eine Umfangsnut 14 als Rast-/Verriegelungsnut ausgeformt.

Der Handgriff 4 besteht gemäß der Fig. 1a und 1b aus einer äußeren Hülse 16, an deren distalem Axialabschnitt das hülsenförmige Kupplungsverriegelungselement 12 (einstückig) ausgeformt oder montiert ist. In einem Mittenabschnitt der äußeren Hülse ist ein manuell betätigbares Stellglied 18 in Form eines Betätigungshebels anscharniert. In einem proximalen Axialabschnitt ist ein Schlauchanschluss 20 ausgebildet, in welchen ein vorzugsweise flexibler Druckschlauch 22 vorzugsweise nach dem Luerlock-Prinzip montiert ist. Der Schlauch 22 ist wiederum an eine in Fig. 8 angedeutete Druckluftquelle 24, beispielsweise in Form eines Kompressors oder einer stationären Druckluft-Ringleitung, anschließbar.

Der Betätigungshebel 18 dient vorliegend für das Ein - Ausschalten (Öffnen - Schließen) des Ventilmechanismus 10 innerhalb des Handgriffs 4 zur wahlweisen Druckbeaufschlagung des angekoppelten sowie verriegelten Instruments/Motoreinheit 1 bzw. 2. Es ist aber auch denkbar, den Betätigungshebel 18 vergleichbar zu einem Gashebel dosiert zu betätigen, um in Abhängigkeit des Betätigungsbetrags die Druckluftzufuhr zu regeln/steuern.

An einer dem Betätigungshebel 18 vorzugsweise diametral gegenüberliegenden Stelle an der Außenhülse 16 und insbesondere an deren Kupplungs-Verriegelungselement/- abschnitt 12 ist ein Druckschalter oder Druckknopf 26 radialverschieblich gelagert.

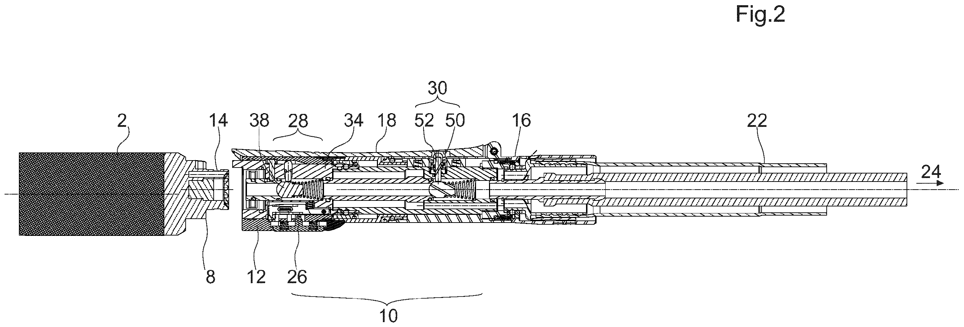

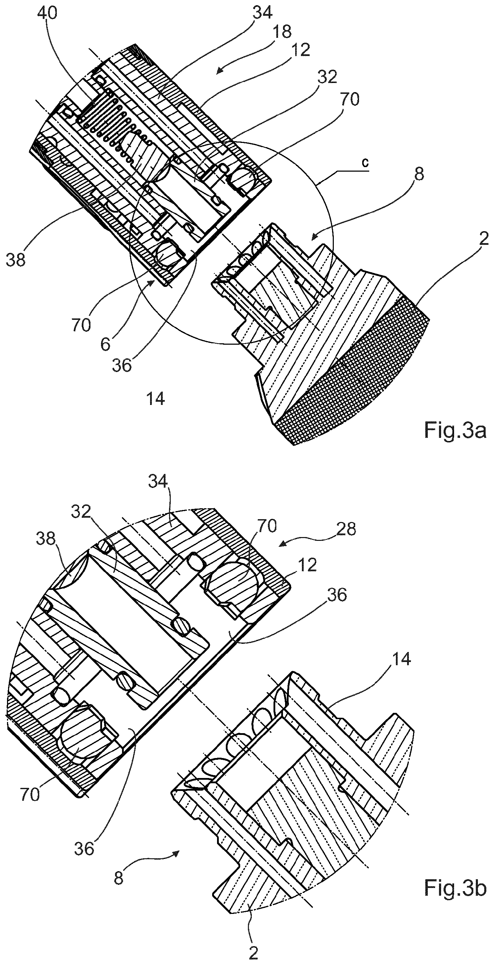

In der Fig. 2 ist das Innenleben (Ventilmechanismus bzw. Regel-/Steuerventil-Mechanismus) des Handgriffs 4 im Überblick dargestellt. Dieses wird nachstehend der Reihe nach von distal in Richtung proximal des Handgriffs 4 unter Verweis auf die jeweils vergrößerten Darstellungen der Fig. 3 bis 7 näher beschrieben.

Der Ventilmechanismus 10 des Handgriffs 4 gemäß dem bevorzugten Ausführungsbeispiel der vorliegenden Erfindung hat eine erste 28 und ein zweite Ventileinrichtung 30, die axial voneinander beabstandet sind. Die erste Ventileinrichtung 28 ist ein ausschließlich über die Kupplung 6 bzw. das Kupplungsverriegelungselement 12 betätigbares Ventil vorzugsweise der Sitzventilbauart, wohingegen die zweite Ventileinrichtung 30 ein über das Stellglied/den Betätigungshebel 18 aktuierbares Ventil vorzugsweise ebenfalls der Sitzventilbauart ist.

Die erste Ventileinrichtung 28 besteht gemäß der Fig. 3a und 3b aus einem hülsenförmigen Ventilgehäuse 32, das axial verschiebbar in einer, von der Außenhülse 16 bzw. dem Kupplungsverriegelungselement 12 relativ bewegbar ummantelte innere Hülse 34 des Handgriffs 4 gelagert ist und an ihrer distalen Stirnseite in einen Aufnahmeschacht 36 für das stutzenförmige, männliche Kupplungsteil 8 auf Seiten des Instruments/Motoreinheit ragt. An der proximalen Stirnseite des Ventilgehäuses 32 ist ein Pilz- oder kugelförmiger Ventilkörper 38 angeordnet, der über eine Kegeldruckfeder 40 gegen das Ventilgehäuse 32 vorgespannt ist und somit das Ventilgehäuse 32 axial abdichtet. Die Kegeldruckfeder 40 stützt sich hierbei gegen eine Ringschulter an der inneren Hülse 34 des Handgriffs 4 ab.

An der äußeren Umfangsfläche des Ventilgehäuses 32 ist wenigstens eine Umfangsnut 42 als Ausweichnut eingearbeitet. Im Bereich der Ausweichnut 42 ist ein Sperrstift 44 vorgesehen, der in der inneren Hülse 34 radial verschiebbar gelagert ist. Der Sperrstift 44 stützt sich an seiner radial inneren Stirnseite an der Umfangsfläche des Ventilgehäuses 32 ab und ist an seiner radial äußeren Stirnseite in einer Axialkulisse am Innumfang des Kupplungsverriegelungselements 12 geführt.

Das Ventilgehäuse 32 und das Kupplungsverriegelungselement 12 sind konstruktiv sowie positionstechnisch so aufeinander abgestimmt, dass dann, wenn das männliche Kupplungsteil 8 in den Aufnahmeschacht 36 des weiblichen Kupplungsteils 6 eingesteckt wird, der eingesteckte Stutzen 8 das Ventilgehäuse 32 gegen die Federvorspannung des Ventilkörpers 38 axial verschiebt, bis der radiale Sperrstift 44 in die Ausweichnut 42 gleitet und dabei das Kupplungsverriegelungselement 12 an deren innere Kulissenführung für eine Axialbewegung freigibt. Wird hingegen der Stutzen 8 aus dem Aufnahmeschacht 36 gezogen, drückt die Federvorspannung durch die Feder 40 das Ventilgehäuse 32 axial ein Stück weit in den Aufnahmeschacht 36 nach, wobei der Sperrstift 44 aus der Ausweichnut 42 radial nach außen verschoben wird und in eine Nut 46 an der inneren Kulissenführung des Kupplungsverriegelungselements 12 einrastet. Damit kann das Kupplungsverriegelungselement 12 in entkoppeltem Zustand der Kupplungsteile 6, 8 nicht mehr axial bezüglich der inneren Hülse 34 des Handgriffs 4 bewegt werden.

In anderen Worten ausgedrückt dient der vorstehend beschriebene Mechanismus dazu, eine Betätigung des Kupplungsverriegelungselements 12 mittels des Sperrstifts 44 dann zu blockieren, wenn der Stutzen 8 nicht in den Aufnahmeschacht 36 eingesteckt ist. Die Verschiebung des Ventilgehäuses 32 zur Aktuierung des Sperrstifts 44 ist hierfür nur eine konstruktive Variante. Denkbar wäre auch, den Sperrstift so zu lagern, dass dieser unmittelbar vom Stutzen 8 bei dessen Einstecken in den Aufnahmeschacht 36 aktuiert wird. Auch andere konstruktive Lösungen sind hier anwendbar.

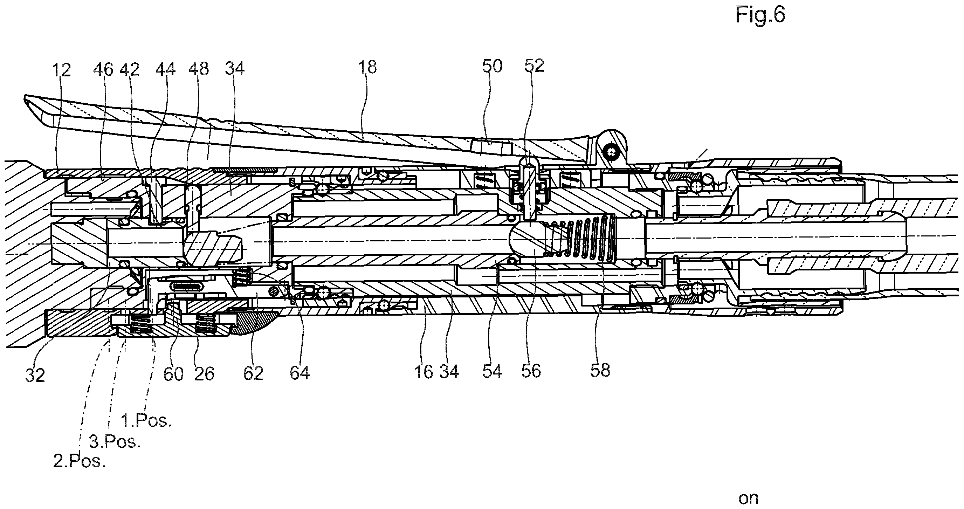

Ferner ist parallel zu dem Sperrstift 44 ein Betätigungsstift 48 in der inneren Hülse 34 radial verschiebbar gelagert, der an seiner inneren Stirnseite am Ventilkörper 32 anliegt und an seiner radial äußeren Stirnseite in der Kulissenführung aufgenommen ist. Diese bildet im Bereich des Betätigungsstifts 48 eine radial nach innen ansteigende Rampe (siehe insbesondere Fig. 6), derart, dass bei einem Verschieben des Kupplungsverriegelungselements 12 in Richtung distal zum Verriegeln der Kupplungsteile 6, 8 der Betätigungsstift 48 radial nach innen bewegt wird und dabei den Ventilkörper 38 der ersten Ventileinrichtung 28 radial vom Ventilsitz des Ventilgehäuses 32 wegdrückt.

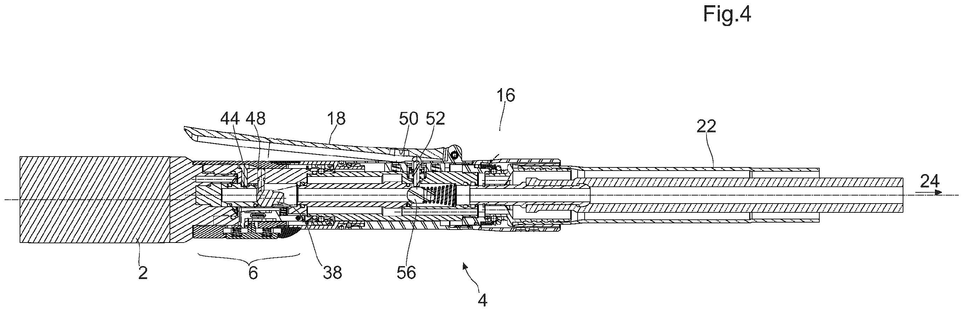

In anderen Worten ausgedrückt, bewirkt ein Verschieben des Kupplungsverriegelungselements 12 aus der Entriegelungsposition gemäß der Fig. 2 in Richtung Verriegelungsposition beispielsweise gemäß der Fig. 4 automatisch bzw. zwangsläufig ein mechanisches Öffnen der ersten Ventileinrichtung 28 über den Betätigungsstift 48.

Wie ferner aus der Fig. 2 zu erkennen ist, wird der Betätigungshebel 18, weil an der äußeren (verschiebbaren) Hülse 16 anscharniert, bei einer Axialbewegung des Kupplungsverriegelungselements 12 als distaler Abschnitt der äußeren Hülse 16 bezüglich der inneren Hülse 34 axial mitbewegt. D.h. der Betätigungshebel 18 verändert bei Betätigen des Kupplungsverriegelungselements 12 automatisch/zwangsläufig seine Axialposition bezüglich der inneren Hülse 34.

Der Betätigungshebel 18 hat an seiner der äußeren Hülse 16 zugewandten Unterseite eine axial sich erstreckende Ausnehmung oder Kerbe (Sackloch) 50. In diesem Axialbereich ist ein weiterer Betätigungsstift 52 radial verschiebbar in der inneren Hülse 16 gelagert. Die Relativlage zwischen Hebel 18 und weiterer Betätigungsstift 52 ist so abgestimmt, dass in einer Entriegelungsposition des Kupplungsverriegelungselements 12 (siehe Fig. 2) der Hebel 18 eine Relativlage einnimmt, in welcher der weitere Betätigungsstift 52 radial in die Kerbe 50 ragt. D.h., wird in dieser Relativlage der Hebel 18 in Richtung hin zur Außenseite der äußeren Hülse 16 verschwenkt, taucht der weitere Betätigungsstift 52 frei in die Ausnehmung/Kerbe 50 am Hebel 18 ein, ohne dass dieser vom Hebel 18 radial verschoben wird. Die Verschwenkung des Hebels 18 ist somit wirkungslos, wie dies beispielsweise in der Fig. 2 angedeutet ist.

Wird hingegen das Kupplungsverriegelungselement 12 in dessen (aktive) Verriegelungsposition axial verschoben, bewegt sich der Hebel 18 automatisch mit. Dabei kommt der weitere Betätigungsstift 52 aus dem Bereich der hebelseitigen Ausnehmung/Kerbe 50, wie dies beispielsweise in der Fig. 4 angedeutet ist. Wird in dieser axialen Relativposition der Hebel 18 in Richtung hin zur äußeren Hülse 16 verschwenkt, drückt dieser den weiteren Betätigungsstift 52 radial nach innen.

Radial innerhalb des weiteren Betätigungsstifts 52 ist die zweite Ventileinrichtung 30 vorgesehen bestehend aus einem hülsenförmigen Ventilgehäuse 54, anderen proximaler Stirnseite ein kugel- oder pilzförmiger Ventilkörper 56 angeordnet ist, der mittels einer (Kegel-) feder 58 axial gegen einen stirnseitigen Ventilsitz am Ventilgehäuse 54 gedrückt wird. Der Ventilkörper 56 ist exakt radial unterhalb des Weiteren Betätigungsstifts 52 positioniert, derart, dass bei dessen Verschiebung radial nach innen der Ventilkörper 56 vom Ventilsitz weggedrückt wird und damit die zweite Ventileinrichtung 30 öffnet.

Schließlich ist im Bereich des Kupplungsverriegelungselements 12 der Betätigungsknopf oder die Taste 26 radial verschiebbar an der äußeren Hülse 16 gelagert.

Der Betätigungskopf 26 hat einen Aktuierungsfinger oder Zapfen 60, der gegen eine schwenkbar an der inneren Hülse 34 gelagerte Rastklinke 62 anliegt. Die Rastklinke 62 wird mittels einer Feder 64 radial nach außen gegen die äußere Hülse 16 vorgespannt, die im Bereich des Kupplungsbetätigungselements/-abschnitts 12 wenigstens zwei, vorzugsweise 3 axial beabstandete Innenbohrungen 1. Pos bis 3. Pos aufweist, in welche die Rastklinke 62 je nach Axialposition der äußeren Hülse 16 relativ zur inneren Hülse 34 federvorgespannt einrastet und so die eingenommene Axialposition fixiert.

Die drei axial beabstandeten Innenbohrungen 1. Pos bis 3. Pos betreffen die einzelnen Betätigungspositionen des Kupplungsverriegelungselements, wie nachfolgend beschrieben:
Die Innenbohrung 1. Pos betrifft gemäß der Fig. 6 jene Betätigungsposition, in welcher die eingekoppelten Kupplungsteile 6, 8 mittels des Kupplungsverriegelungselements 12 verriegelt sind und gleichzeitig die erste Ventileinrichtung 26 geöffnet und die zweite Ventileinrichtung 30 freigeschaltet ist. Diese Position wird als aktive Verriegelungsposition bezeichnet.

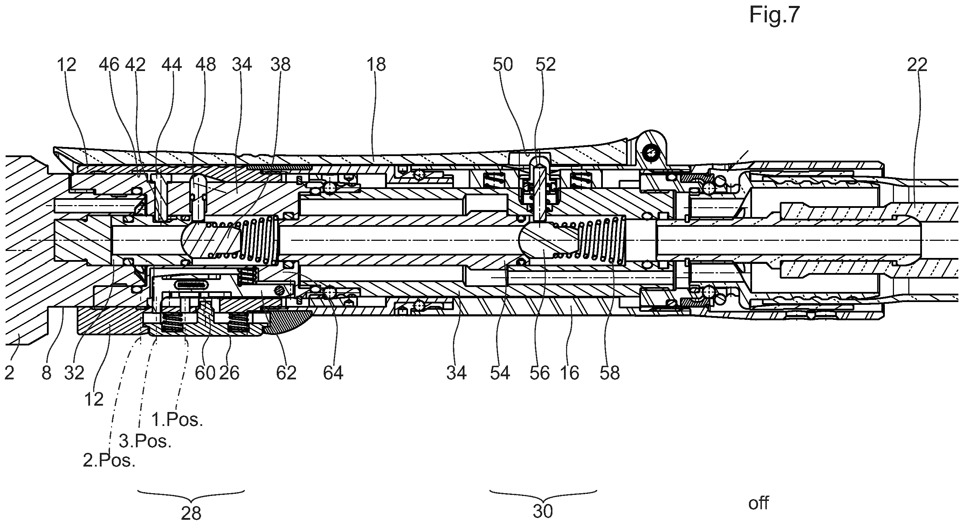

Die Innenbohrung 3. Pos betrifft gemäß der Fig. 7 jene Betätigungsposition, in welcher die eingekoppelten Kupplungsteile 6, 8 mittels des Kupplungsverriegelungselements 12 verriegelt sind und gleichzeitig die erste Ventileinrichtung 26 geöffnet und die zweite Ventileinrichtung 30 jedoch nicht freigeschaltet ist. D.h. der weitere Betätigungsstift 52 befindet sich noch immer innerhalb der Ausnehmung/Kerbe 50 im Fall eines Verschwenkens des Hebels 18. Diese Position wird als passive Verriegelungsposition bezeichnet, da die Kupplung verriegelt, der Ventilmechanismus 10 aber nicht aktuierbar ist. Die passive Verriegelungsposition stellt optional auch jene Position dar, die bei Drücken des Betätigungsknopfs 26 aus der aktiven Verriegelungsposition kommend, vorzugsweise selbsttätig durch eine axiale Federvorspannung eingenommen wird, um die Druckluftzufuhr im Notfall zu unterbrechen, ohne dass der Hebel 18 freigegeben werden muss. Die passive Verriegelungsposition kann daher auch als Not - Aus - Position bezeichnet werden.

Die Innenbohrung 2. Pos betrifft gemäß der Fig. 2 jene Betätigungsposition, in welcher die Kupplungsteile 6, 8 unverriegelt sind, wobei die erste Ventileinrichtung 26 geschlossen und die zweite Ventileinrichtung 30 nicht freigeschaltet ist.

An dieser Stelle sei darauf hingewiesen, dass die Innenbohrung 3. Pos eine optionale vorteilhafte Maßnahme darstellt. Der erfindungsgemäße Handgriff 4 könnte theoretisch seine Grundfunktionen auch ohne diese Innenbohrung ausführen. Ferner sei darauf hingewiesen, dass alle jene Merkmale, die eingangs der Beschreibung zu jenem Stand der Technik aufgelistet wurden, welcher erfindungsgemäß weitergebildet werden soll, auch bei der vorliegenden Erfindung technisch umgesetzt sind, sodass auf deren erneute Beschreibung an dieser Stelle verzichtet werden kann.

Nachstehend wird die Funktion des erfindungsgemäßen medizinischen Handgriffs bzw. des erfindungsgemäßen medizinischen Instrumentensystems näher beschrieben.

Zunächst wird der Druckschlauch 22 mit daran montiertem Handgriff 4 an die Druckmittelquelle 24 angeschlossen, sodass der Handgriff 4 unmittelbar darauf Druckbeaufschlagt ist. In dieser Phase ist die äußere Hülse 16 bezüglich der inneren Hülse 34 des Handgriffs 4 in Richtung proximal verschoben, wobei die Rastklinke 62 in die Innenbohrung 2. Pos am Kupplungsverriegelungselement 12 federvorgespannt eingerastet ist. Damit nimmt der Handgriff 4 die Entriegelungsposition ein, in welcher die erste Ventileinrichtung 26 geschlossen und die zweite Ventileinrichtung 30 nicht freigeschaltet ist. Diese Position wird zudem durch den Sperrstift 44 fixiert, welcher mittels des Ventilgehäuses 32 der ersten Ventileinrichtung 28 radial nach außen in die Innennut 46 der Kupplungsverriegelungselements 12 gedrückt ist. In dieser Betriebsposition kann das Kupplungsverriegelungselement 12 nicht axial verschoben werden. Eine Betätigung des Hebels 18 ist zwar möglich, jedoch wirkungslos, da der weitere Betätigungsstift 52 in die Ausnehmung/Kerbe 50 am Hebel 18 eintauchen würde und daher nicht axial verschoben werden kann.

Selbst wenn der Hebel 18 von der Außenhülse 16 wegverschwenkt oder abgebrochen werden würde, sodass eine unmittelbare manuelle Betätigung des Weiteren Betätigungsstifts 52 möglich wäre, kann die Funktionssicherheit nach wie vor garantiert werden, da die ersten Ventileinrichtung 28 in jedem Fall geschlossen ist.

Sobald das Instrument 1 oder dessen separate Motoreinheit (Antriebszelle) 2 über deren Kupplungsstutzen 8 in den Aufnahmeschacht 36 der Handgriffseitigen Kupplung 6 eingesteckt ist, wird dadurch das Ventilgehäuse 32 der ersten Ventileinrichtung 28 axial verschoben, wobei der Sperrstift 44 aus der Innennut 46 des Kupplungsverriegelungselements 12 in die Ausweichnut 42 am Ventilgehäuse 32 gleitet und so das Kupplungsverriegelungselement 12 für dessen manuelle Axialverschiebung freigibt. Jetzt kann das Kupplungsverriegelungselement 12 axial in Richtung distal verschoben werden, bis die an der Innenhülse 34 schwenkgelagerte Sperrklinke 62 in die Innenbohrung 2. Pos einrastet und die äußere Hülse 16 bzw. das Kupplungsverriegelungselement 12 in der aktiven Verriegelungsposition fixiert.

In dieser Position ist der Betätigungsstift 4 der ersten Ventileinrichtung 28 vom Kupplungsverriegelungselement 12 radial nach innen verschoben du drückt den Ventilkörper 38 der ersten Ventileinrichtung 28 vom Ventilsitz weg. Die erste Ventileinrichtung ist somit geöffnet. Gleichzeitig ist der Betätigungshebel/Stellglied 18 in Richtung distal mit verschoben, derart, dass der weitere Betätigungsstift 52 der zweiten Ventileinrichtung 30 aus dem Bereich der Ausnehmung/Kerbe 50 im Hebel 18 gerät. Wird nunmehr der Hebel 18 gegen die Außenhülse 16 des Handgriffs 4 verschwenkt, kommt der Hebel 18 an der radial äußeren Stirnfläche des Weiteren Betätigungsstifts 52 in Anlage. Ei weiterem Verschwenken des Betätigungshebels 18 verschiebt sich der weitere Betätigungsstift 52 radial nach innen du drückt den Ventilkörper 56 der zweiten Ventileinrichtung 30 von dessen Ventilsitz weg. Damit ist auch die zweite Ventileinrichtung 30 geöffnet und das Instrument/Motoreinheit wird mit Druckmittel beaufschlagt.

Die zweite Ventileinrichtung kann dabei ein Auf- Zu - Ventil sein oder ein Dosierventil, welches die Druckmittelmenge pro Zeiteinheit in Abhängigkeit des Betätigungsgrads des Hebels 18 regelt. Alternativ zum gezeigten Sitzventil sind für die zweite Ventileinrichtung aber ggf. auch für die erste Ventileinrichtung ein Schiebeventil denkbar.

Um das Instrument wieder abkoppeln zu können, muss zunächst der Hebel 18 freigegeben werden, wobei sich die zweite Ventileinrichtung schließt. Hierauf wird der Druckknopf 26 gedrückt, sodass die Rast-/Sperrklinke aus der Innenbohrung 2. Pos an der Außenhülse 16 bzw. am Kupplungsverriegelungselement 12 ausrastet und das Kupplungsverriegelungselement 12 für eine Entriegelungsbewegung freigibt. Wird nunmehr das Kupplungsverriegelungselement 12 axial in Richtung proximal verschoben, greift die Rastklinke 62 bei Erreichen der Entkopplungsposition in die Innenbohrung 1. Pos ein und fixiert dort das Kupplungsverriegelungselement 12. In dieser Position ist der Betätigungsstift 48 freigegeben und der Ventilkörper 38 auf seinen Ventilsitz zurückgekehrt. Die erste Ventileinrichtung 28 ist damit geschlossen.

Wird nunmehr der Stutzen 8 des Instruments/Motoreinheit 2 aus dem Aufnahmeschacht 36 gezogen, wandert das Ventilgehäuse 32 der ersten Ventileinrichtung 28 ein Stück weit in den Aufnahmeschacht 32 federvorgespannt nach und drückt dabei den Sperrstift 44 in die Innennut 46 am Kupplungsverriegelungselement 12. Damit wird das Kupplungsverriegelungselement 12 axialverriegelt, sodass die erste Ventileinrichtung 28 nicht mehr geöffnet werden kann.

In einem Notfall bei betätigtem Hebel 18, kann der Druckknopf 26 gedrückt werden, worauf die Rastklinke 62 aus der Innenbohrung 2. Pos gedrückt und das Kupplungsverriegelungselement 12 ggf. selbsttätig durch eine geeignete axiale Federvorspannung (nicht weiter gezeigt) oder durch manuelles Verschieben in Richtung proximal bewegt wird, bis die Rastklinke 62 federvorgespannt in die (axial mittlere) Innenbohrung 3. Pos einschnappt und dort das Kupplungsverriegelungselement 12 fixiert. In dieser Betätigungsposition (passive Verriegelungsposition) ist die Kupplung noch immer verriegelt, jedoch der Hebel 18 bezüglich der Innenhülse 34 soweit verschoben, dass der weitere Betätigungsstift 52 bereits in die Ausnehmung/Kerbe 50 am Hebel 18 gleitet und daher über den Hebel nicht mehr verschoben werden kann.

Abschließend sei noch auf den Kupplungsverriegelungsmechanismus hingewiesen, welcher vom Kupplungsverriegelungselement 12 betätigbar ist.

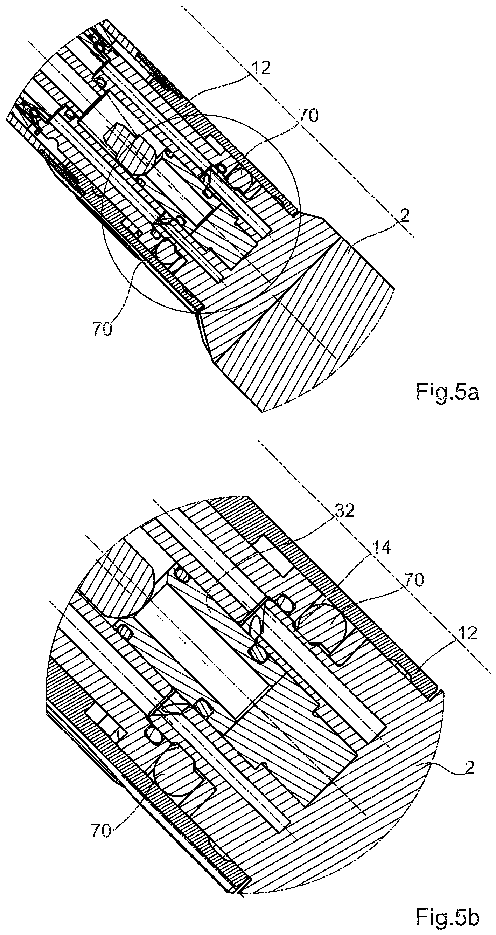

Im einfachsten Fall handelt es sich bei dem Kupplungsverriegelungsmechanismus beispielsweise gemäß der Fig. 3a, 3b oder 5a, 5b um die Anordnung mehrerer (vorzugsweise vier) Rastelemente 70 (Kugeln, Stifte, etc.), die in Radialbohrungen an der Innenhülse 34 radial bewegbar gelagert sind, welche wiederum in Umfangsrichtung beabstandet sind. Diese Rastelemente 70 sind radial nach innen federvorgespannt und ragen in den Aufnahmeschacht 32 etwas vor.

Wird daher der Stutzten 8 am Instrument/Motoreinheit 2 in den Aufnahmeschacht 32 eingesteckt, werden die Rastelemente 70 zunächst radial nach außen verdrängt und schnappen schließlich in die Nut 14 am Stutzen 8 federvorgespannt ein.

In dieser Betriebsposition wird der Stutzen 8 bereit im Aufnahmeschacht 32 gehalten, wobei die Kupplungsverbindung jedoch nicht kraftbelastbar ist. D.h. der Stutzen 8 kann unter Überwindung der Federvorspannung auf die Rastelemente 70 wieder aus dem Aufnahmeschacht 32 gezogen werden. Sobald jedoch das Kupplungsverriegelungselement 12 in Richtung distal verschoben wurde, übergreift dieses die Rastelemente am Außenumfang der Innenhülse 34 und verhindert so eine Verdrängungsbewegung des Rastelement 70 radial nach außen. Die Kupplung ist nun verriegelt.

## Patentansprüche

1. Medizinischer Handgriff für pneumatisch oder hydraulisch angetriebene medizinische Instrumente mit einem handgriffseitigen Kupplungsteil (6) zur mechanischen und pneumatisch-/hydraulischen Ankupplung eines entsprechenden, instrumentenseitigen Kupplungsteils (8) und einem manuell betätigbaren Ventilmechanismus (10) zur wahlweisen Druckbeaufschlagung eines daran angekuppelten medizinischen Instruments (1) oder dessen Motoreinheit (2), der mit einer Sicherungsfunktion ausgestattet ist zur Verhinderung einer Druckbeaufschlagung im Fall eines abgekoppelten medizinischen Instruments (1) oder dessen Motoreinheit (2), wobei das handgriffseitige Kupplungsteil (6) mit einer ersten Ventileinrichtung (28) des Ventilmechanismus (10) versehen ist oder mit dieser zusammenwirkt und das handgriffseitige Kupplungsteil (6) mit einer zweiten, manuell zu betätigenden Ventileinrichtung (30) des Ventilmechanismus (10) zusammenwirkt,
das handgriffseitige Kupplungsteil (6) mit dem Ventilmechanismus (10) zusammenwirkt, derart, dass die erste Ventileinrichtung (30) beim oder durch den mechanischen Kopplungsvorgang beim druckfesten Verriegeln des handgriffseitigen Kupplungsteils (6) automatisch geöffnet wird und
die zweite Ventileinrichtung (30) nur im gekoppelten und vorzugsweise verriegelten Zustand des handgriffseitigen Kupplungsteils (6) für ein manuelles Öffnen und Schließen funktionsfähig ist, so dass das handgriffseitige Kupplungsteil (6) mit dem Ventilmechanismus (10) zusammenwirkt, derart, dass die zweite Ventileinrichtung (30) beim oder durch den mechanischen Kopplungsvorgang für ein manuelles Öffnen freigeschaltet wird, wohingegen
im abgekoppelten Zustand dementsprechend die erste Ventileinrichtung (30) des Ventilmechanismus (10) geschlossen ist und die zweite Ventileinrichtung (30) funktionsunfähig und dementsprechend die Freischaltung der zweiten Ventileinrichtung (30) aufgehoben ist.

2. Medizinischer Handgriff nach Anspruch 1, **dadurch gekennzeichnet, dass** der Ventilmechanismus (10) ein manuell betätigbares, vorzugsweise hebeiförmiges Stellglied (18) hat, welches direkt oder indirekt auf die zweite Ventileinrichtung (30) für deren wahlweises Öffnen einwirkt, wobei durch oder mit Verriegeln des Kupplungsteils (6) auf Seiten des Handstücks (4) automatisch die Funktionsfähigkeit des vorzugsweise hebelförmigen Stellglieds (18) hergestellt wird und wenigstens durch oder mit Entriegeln des Kupplungsteils (6) das Stellglied (18) außer Funktion gesetzt wird.

3. Medizinischer Handgriff nach Anspruch 2, **dadurch gekennzeichnet, dass** ein bewegbar am Handgriff (4) gelagertes, manuell betätigbares Kupplungsverriegelungselement (12) des Kupplungsteils (6) mechanisch mit der ersten Ventileinrichtung (28) und/oder mechanisch mit dem Stellglied (18) der zweiten Ventileinrichtung (18) wirkverbunden ist, sodass ein manuelles Betätigen des Kupplungsverriegelungselements (12) automatisch ein Öffnen/Schließen der ersten Ventileinrichtung (28) bewirkt und/oder eine entsprechende Wirkung auf die Funktionsfähigkeit des Stellglieds (18) ausübt.

4. Medizinischer Handgriff nach Anspruch 3, **dadurch gekennzeichnet, dass** das Stellglied (18) bei/durch Entriegeln des Kupplungsteils (6) mittels des Kupplungsverriegelungselements (28) in eine Position mitbewegt wird, in welcher die Wirkverbindung mit der zweiten Ventileinrichtung (30) unterbrochen ist oder dass bei Entriegeln des Kupplungsteils (6) durch das Kupplungsverriegelungselement (28) eine Sperre in den Betätigungsweg des Stellglieds (18) gefahren wird.

5. Medizinischer Handgriff nach Anspruch 3 oder 4, **dadurch gekennzeichnet, dass** für das Kupplungsverriegelungselement (12) mehrere Betätigungspositionen (1. Pos bis 3. Pos) vorgesehen sind, vorzugsweise eine ersten Betätigungsposition (1. Pos) als Entriegelungsposition für ein Abkoppeln des Handgriffs (4) vom Instrument/Motoreinheit (1, 2), eine zweite Betätigungsposition (2. Pos) als aktive Verriegelungsposition für ein Verriegeln der gekoppelten Kupplungsteile (6, 8) bei gleichzeitigem Öffnen der ersten Ventileinrichtung (28) und in Funktion setzen der zweiten Ventileinrichtung (30) und vorzugsweise einer dritten Betätigungsposition (3. Pos) als passive Verriegelungsposition für ein Verriegeln der Kupplungsteile (6, 8) bei gleichzeitigem Öffnen der ersten Ventileinrichtung (28) und außer Funktion setzen der zweiten Ventileinrichtung (30).

6. Medizinischer Handgriff nach Anspruch 5, **gekennzeichnet durch** einen Druckknopf oder Schalter (26) im Bereich des Kupplungsverriegelungselements (12), bei dessen Betätigung das Kupplungsverriegelungselement (12) vorzugsweise selbsttätig aufgrund einer axialen Vorspannung aus der zweiten Betätigungsposition (2. Pos) in die dritte Betätigungsposition verlagert wird.

7. Medizinischer Handgriff nach Anspruch 6, **dadurch gekennzeichnet, dass** die dritte Betätigungsposition (3. Pos) im Betätigungsweg zwischen der ersten und zweiten Betätigungsposition liegt.

8. Medizinische Instrumentensystem mit einem hydraulisch oder pneumatisch betriebenen medizinischen Instrument (1) oder dessen Motoreinheit (2), woran ein vorzugsweise männliches Kupplungsteil (8) zur mechanischen und hydraulisch/pneumatischen Kupplung mit einem vorzugsweise weiblichen Kupplungsteil (6) an einem Handstück (4) angeordnet ist, das über einen Druckschlauch (22) mit einer Druckquelle (24) verbindbar ist, **dadurch gekennzeichnet, dass** der Handgriff (4) die Merkmale eines der Ansprüche 1 bis 7 aufweist.

9. Medizinischen Instrumentensystem nach Anspruch 8, **dadurch gekennzeichnet, dass** das männliche Kupplungsteil (8) bei Einstecken in das weibliche Kupplungsteil (6) automatisch ein Rastelement (44) betätigt für ein Freigeben des Kupplungsverriegelungselements (12) zur wahlweisen manuellen Verriegelung des weiblichen Kupplungsteils (6) bei eingekoppeltem männlichen Kupplungsteil (8).

## Claims

1. A medical handle for pneumatically or hydraulically driven medical instruments comprising a handle-side clutch member (6) for mechanical and pneumatic/hydraulic coupling of a corresponding instrument-side clutch member (8) and a manually operable valve mechanism (10) for selective pressurization of a medical instrument (1) coupled thereto or the motor unit (2) thereof, said handle being equipped with a safety function for preventing pressurization in the case of an uncoupled medical instrument (1) or the motor unit (2) thereof, **wherein**
the handle-side clutch member (6) is provided with a first valve device (28) of the valve mechanism (10) or interacts with the same and the handle-side clutch member (6) interacts with a second manually-operated valve device (30) of the valve mechanism (10),
the handle-side clutch member (6) interacts with the valve mechanism (10) such that during or by the mechanical coupling operation the first valve device (30) is automatically opened during pressure-tight locking of the handle-side clutch member (6) and
the second valve device (30) is operative for manual opening and closing only in the coupled and preferably locked state of the handle-side clutch member (6), so that the handle-side clutch member (6) interacts with the valve mechanism (10) such that during or by the mechanical coupling operation the second valve device (30) is enabled for manual opening, whereas
in the uncoupled state, correspondingly, the first valve device (30) of the valve mechanism (10) is closed and the second valve device (30) is functional and correspondingly enabling of the second valve device (30) is cancelled.

2. The medical handle according to claim 1, **characterized in that** the valve mechanism (10) includes a manually operable, preferably lever-type actuator (18) which directly or indirectly acts on the second valve device (30) for selective opening thereof, wherein by or upon locking the clutch member (6) on the side of the handle (4) the functioning of the preferably lever-type actuator (18) is automatically brought about and at least by or upon unlocking the clutch member (6), the actuator (18) is put out of function.

3. The medical handle according to claim 2, **characterized in that** a manually operable clutch-locking element (12) of the clutch member (6) being movably supported on the handle (4) is operatively connected mechanically to the first valve device (28) and/or mechanically to the actuator (18) of the second valve device (28) so that manual operation of the clutch-locking element (12) automatically causes the first valve device (28) to open/close and/or exerts an appropriate effect on the functioning of the actuator (18).

4. The medical handle according to claim 3, **characterized in that** the actuator (18) is moved along upon/by unlocking the clutch member (6) by means of the clutch-locking element (28) to a position in which the operative connection to the second valve device (30) is disconnected, or **in that** upon unlocking the clutch member (6) by the clutch-locking element (28) a stop is moved into the operating path of the actuator (18).

5. The medical handle according to claim 3 or 4, **characterized in that** for the clutch-locking element (12) plural operating positions (position 1 to position 3) are provided, preferably a first operating position (pos. 1) as an unlocking position for uncoupling the handle (4) from the instrument/motor unit (1, 2), a second operating position (pos. 2) as an active locking position for locking the coupled clutch members (6, 8) while simultaneously opening the first valve device (28) and putting the second valve device (30) into function, and preferably a third operating position (pos. 3) as a passive locking position for locking the clutch members (6, 8) while simultaneously opening the first valve device (28) and putting the second valve device (30) out of function.

6. The medical handle according to claim 5, **characterized by** a push button or switch (26) in the area of the clutch-locking element (12) upon actuation of which the locking element (12) is shifted preferably automatically due to axial bias from the second operating position (pos. 2) to the third operating position.

7. The medical handle according to claim 6, **characterized in that** the third operating position (pos. 3) is located in the operating path between the first and second operating positions.

8. A medical instrument system comprising a hydraulically or pneumatically operated medical instrument (1) or the motor unit (2) thereof on which a preferably male clutch member (8) is arranged for mechanical and hydraulic/pneumatic coupling to a preferably female clutch member (6) on a handle (4) which is adapted to be connected to a pneumatic source (24) via a pneumatic hose (22), **characterized in that** the handle (4) comprises the features of any one of the claims 1 to 7.

9. The medical instrument system according to claim 8, **characterized in that** upon insertion in the female clutch member (6) the male clutch member (8) automatically actuates a stop element (44) for releasing the clutch-locking element (12) for selective manual locking of the female clutch member (6) when the male clutch member (8) is coupled.

## Revendications

1. Poignée à usage médical destinée à des instruments médicaux à entraînement pneumatique ou hydraulique munie d'une partie de couplage côté poignée (6) pour le couplage mécanique et pneumatique/hydraulique d'une partie de couplage correspondante côté instrument (8) et un mécanisme à soupape actionnable manuellement (10) destiné à mettre sous pression de manière sélective un instrument médical (1) ou son unité motrice (2) couplé(e) à celle-ci, qui est équipée d'une fonction de sécurité pour empêcher une mise sous pression en cas d'un instrument médical (1) ou de son unité motrice (2) découplé(e),
la partie de couplage côté poignée (6) étant pourvue d'un premier dispositif formant soupape (28) du mécanisme à soupape (10) ou coopérant avec celui-ci et la partie de couplage côté poignée (6) coopérant avec un deuxième dispositif formant soupape (30) à actionner manuellement du mécanisme à soupape (10),
la partie de couplage côté poignée (6) coopérant avec le mécanisme à soupape (10), de sorte que le premier dispositif formant soupape (30) est ouvert automatiquement lors de ou par l'opération de couplage mécanique lors du verrouillage résistant à la pression de la partie de couplage côté poignée (6) et
le deuxième dispositif de soupape (30) étant uniquement fonctionnel à l'état couplé et de préférence verrouillé de la partie de couplage côté poignée (6) pour une ouverture et une fermeture manuelles, de sorte que la partie de couplage côté poignée (6) coopère avec le mécanisme à soupape (10), de sorte que le deuxième dispositif formant soupape (30) lors ou par l'opération de couplage mécanique est déverrouillé pour une ouverture manuelle, tandis qu'à
l'état découplé en conséquence le premier dispositif formant soupape (30) du mécanisme à soupape (10) étant fermé et le deuxième dispositif formant soupape (30) étant fonctionnel et en conséquence le déverrouillage du deuxième dispositif formant soupape (30) étant supprimé.

2. Poignée à usage médical selon la revendication 1, **caractérisée en ce que** le mécanisme à soupape (10) présente un actionneur (18) actionnable manuellement, de préférence sous forme de levier, lequel fonctionne directement ou indirectement sur le deuxième dispositif formant soupape (30) pour leur ouverture sélective, par ou grâce au verrouillage de la partie de couplage (6) du côté de la pièce à main (4) la capacité fonctionnelle de l'actionneur (18) de préférence sous forme de levier étant établie automatiquement et au moins par ou grâce au déverrouillage de la partie de couplage (6) l'actionneur (18) est désactivé.

3. Poignée à usage médical selon la revendication 2, **caractérisée en ce qu'**un élément de verrouillage du couplage (12) actionnable manuellement monté de manière mobile sur la poignée (4) de la partie de couplage (6) est relié fonctionnellement de manière mécanique au premier dispositif formant soupape (28) et/ou de manière mécanique à l'actionneur (18) du deuxième dispositif formant soupape (18), de sorte qu'un actionnement manuel de l'élément de verrouillage du couplage (12) induit automatiquement une ouverture/fermeture du premier dispositif formant soupape (28) et/ou exerce une action correspondante sur la capacité fonctionnelle de l'actionneur (18).

4. Poignée à usage médical selon la revendication 3, **caractérisée en ce que** l'actionneur (18) lors du/par le déverrouillage de la partie de couplage (6) au moyen de l'élément de verrouillage du couplage (28) est déplacé dans une position, dans laquelle la liaison fonctionnelle avec le deuxième dispositif formant soupape (30) est rompue ou **en ce que** lors du déverrouillage de la partie de couplage (6) par l'élément de verrouillage du couplage (28) un verrou est enclenché lors de la course d'actionnement de l'actionneur (18).

5. Poignée à usage médical selon la revendication 3 ou 4, **caractérisée en ce que** plusieurs positions d'actionnement (pos. 1 à pos. 3) sont prévues pour l'élément de verrouillage du couplage (12), de préférence une première position d'actionnement (pos. 1) en tant que position de déverrouillage pour un découplage de la poignée (4) de l'instrument/unité motrice (1, 2), une deuxième position d'actionnement (pos. 2) en tant que position de verrouillage active pour un verrouillage de la partie de couplage couplée (6, 8) lors de l'ouverture simultanée du premier dispositif formant soupape (28) et activation du deuxième dispositif formant soupape (30) et de préférence une troisième position d'actionnement (pos. 3) en tant que position de verrouillage passive pour un verrouillage de la partie de couplage (6, 8) lors de l'ouverture simultanée du premier dispositif formant soupape (28) et désactivation du deuxième dispositif formant soupape (30).

6. Poignée à usage médical selon la revendication 5, **caractérisée par** un bouton-poussoir ou un commutateur (26) dans la zone de l'élément de verrouillage du couplage (12), lors de son actionnement l'élément de verrouillage du couplage (12) est déplacé de préférence automatiquement en raison d'une précontrainte axiale de la deuxième position d'actionnement (pos. 2) à la troisième position d'actionnement.

7. Poignée à usage médical selon la revendication 6, **caractérisée en ce que** la troisième position d'actionnement (pos. 3) se situe dans la course d'actionnement entre la première et la deuxième position d'actionnement.

8. Système d'instruments médicaux muni d'un instrument médical (1) à entraînement hydraulique ou pneumatique ou son unité motrice (2), auquel est disposé une partie de couplage de préférence mâle (8) pour le couplage mécanique et hydraulique/pneumatique avec une partie de couplage de préférence femelle (6) sur une poignée (4), qui peut être reliée par l'intermédiaire d'un tuyau de pression (22) à une source de pression (24), **caractérisé en ce que** la poignée (4) présente les caractéristiques selon l'une quelconque des revendications 1 à 7.

9. Système d'instruments médicaux selon la revendication 8, **caractérisé en ce que** la partie de couplage mâle (8) lors du branchement dans la partie de couplage femelle (6) actionne automatiquement un élément d'encliquetage (44) permettant une libération de l'élément de verrouillage du couplage (12) pour le verrouillage manuel sélectif de la partie de couplage femelle (6) lors de la partie de couplage mâle couplée (8).
